(19) **Europäisches Patentamt**
**European Patent Office**
**Office européen des brevets**

(11) **EP 2 823 301 B1**

(12) **EUROPEAN PATENT SPECIFICATION**

(45) Date of publication and mention
of the grant of the patent:
**23.08.2017 Bulletin 2017/34**

(51) Int Cl.:
*G01N 33/50* (2006.01)

(21) Application number: **13710809.8**

(22) Date of filing: **07.03.2013**

(86) International application number:
**PCT/EP2013/054665**

(87) International publication number:
**WO 2013/132036 (12.09.2013 Gazette 2013/37)**

(54) **OPTIMAL GONADOTROPIN DOSAGE**

OPTIMALE GONADOTROPINDOSIERUNG

DOSAGE OPTIMAL DE LA GONADOTROPHINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.03.2012 GB 201204013**

(43) Date of publication of application:
**14.01.2015 Bulletin 2015/03**

(73) Proprietor: **Oslo Universitetssykehus HF
0424 Oslo (NO)**

(72) Inventors:
• **FEDORCSAK, Peter
0590 Oslo (NO)**
• **RAKI, Melinda
0590 Oslo (NO)**

(74) Representative: **Dzieglewska, Hanna Eva
Dehns
St Bride's House
10 Salisbury Square
London EC4Y 8JD (GB)**

(56) References cited:
**US-A1- 2003 130 592**

• **KATABUCHI HIDETAKA ET AL: "Human
chorionic villous macrophages as a fetal
biological shield from maternal chorionic
gonadotropin", DEVELOPMENT GROWTH &
DIFFERENTIATION, vol. 50, no. 5, June 2008
(2008-06), pages 299-306, XP002696319, ISSN:
0012-1592**
• **ANDERSEN A NYBOE ET AL: "Predictive factors
of ovarian response and clinical outcome after
IVF/ICSI following a rFSH/GnRH antagonist
protocol with or without oral contraceptive
pre-treatment", HUMAN REPRODUCTION
(OXFORD), vol. 26, no. 12, December 2011
(2011-12), pages 3413-3423, XP002696320,**
• **ASSAF BEN-MEIR ET AL: "The value of human
chorionic gonadotropin stimulation test in
predicting ovarian response during in-vitro
fertilization", JOURNAL OF ASSISTED
REPRODUCTION AND GENETICS, KLUWER
ACADEMIC PUBLISHERS-PLENUM
PUBLISHERS, NE, vol. 28, no. 10, 21 July 2011
(2011-07-21) , pages 893-899, XP019973033,
ISSN: 1573-7330, DOI:
10.1007/S10815-011-9612-Y**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a method of predicting the response of a subject to ovarian stimulation with an exogenous gonadotropin and to the use of such a method in determining the dose, particularly the optimum dose, of gonadotropin to be administered to the subject for ovarian stimulation in assisted reproduction treatment. Optimal gonadotropin dosage depends on age, body weight, and infertility diagnosis, but prediction of individual clinical response is difficult. We propose a new method for estimating an optimal gonadotropin dosage, based on new observations on gonadotropin sequestration by peripheral monocytes *in vitro.*

In a spontaneous menstrual cycle, a single ovarian follicle is generally selected for growth and releases an oocyte that may eventually be fertilized. In situations of compromised fertility, however, ovulation may not occur and may need to be artificially stimulated. In such an ovulation induction treatment, it is generally desired to induce development of only a single ovarian follicle and multiple ovulation is undesired in view of the risks of multiple pregnancy. However, during other forms of assisted reproduction treatment, such as *in vitro* fertilisation (IVF), growth of multiple follicles is desirable in order to compensate for a limited efficiency of fertilization and embryo development *in vitro.* Indeed, approximately one of four aspirated oocytes would yield an embryo of adequate quality for being replaced to the uterus or cryopreserved for subsequent transfer.

Development of multiple follicles is usually induced by exogenous gonadotropin stimulation. The pituitary gonadotropin hormones FSH and LH regulate ovarian function by activating cognate receptors on granulosa and theca cells, respectively. By inducing proliferation of granulosa cells and estradiol synthesis during the early follicular phase, FSH is essential for inducing and supporting growth of follicles, and therefore FSH treatment is an integral component of stimulation protocols.

Prediction of an optimal gonadotropin dosage, and particularly an optimal FSH dosage, during assisted reproduction treatment is a considerable clinical challenge (Fauser, et al., 2008, Hum Reprod Update, 14, 1-14). Initial FSH injections during the early follicular phase are thought to determine the number of growing follicles, whereas maintenance doses influence the pace of follicle growth and follicle health. Insufficiently low gonadotropin doses cannot support recruitment and growth of follicles, whereas higher-than-necessary doses carry the risk of ovarian hyperstimulation syndrome. Furthermore, excess gonadotropin doses are related to an inferior treatment outcome. Indeed, cumulative FSH dosage appears to be an independent predictor of pregnancy and live birth during assisted reproduction treatment, irrespective of age, ovarian response, and other clinical predictors of success. Indeed, it has been reported that more chromosomally normal embryos are available during ovarian stimulation with reduced gonadotropin doses, so-called mild stimulation, compared to conventional regimens. Furthermore, oocytes exposed to increased FSH levels *in vitro* display a higher incidence of aneuploidy, and embryos derived after superovulation have increased incidence of abnormal gene imprinting.

Optimal FSH dosage is thought to be related to age, antral follicle count, baseline FSH concentration, body size and obesity, infertility diagnosis, smoking status, etc. Prediction models that are based on these variables are nonetheless of limited clinical value and may perform poorly at the extremes of gonadotropin response, such as high-responder patients with polycystic ovary syndrome (PCOS) or poor responders with diminished ovarian reserve.

Novel predictive strategies search for factors that either give a more precise estimate of ovarian reserve, like assessment of anti-Müllerian hormone and antral follicle count (Broer, et al., 2011 Human Reproduction Update, 17, 1, 46-54), combination of endocrine tests (Al-Azemi, et al., 2011, Human Reproduction, 26, 414-422), or investigate gonadotropin action on a molecular level, such as studies on genetic variability of hormone receptor genes (Altmäe, et al., 2011 Human Reproduction Update, 17, 813-828).

Clinical response to gonadotropins, however, also varies in successive cycles of an individual woman. Reasons for temporarily changing sensitivity to gonadotropins has been poorly understood, and may indicate that gonadotropin response is largely determined by variable rather than constitutive factors.

A particular problem of gonadotropin dosage occurs in obesity, since gonadotropin pharmacokinetics are related partly to body size. Indeed, overweight and obese women require increased gonadotropin doses during ovarian stimulation, which often exerts a suboptimal ovarian response. Relative gonadotropin resistance in obesity, however, has been difficult to conceptualize. Exogenous gonadotropins achieve lower serum concentrations in obese compared to normal weight women, but altered pharmacokinetics would still be counter-intuitive, since water-soluble hormones are not expected to accumulate or be sequestered in fat. Alternatively, metabolic alterations in obesity, such as insulin resistance, increased insulin concentrations, or increased leptin concentrations, may directly or indirectly interfere with gonadotropin action.

It has been shown that tissue macrophages are capable of internalizing human chorionic gonadotropin (hCG) and may protect fetal gonadogenesis from excess hCG (Katabuchi and Ohba, 2008 Develop. Growth Differ. 50, 299-306). Based on experiments reported in the Examples below, we have determined that gonadotropins may be sequestered in certain cells, and propose that such sequestration may regulate gonadotropin availability and hence gonadotropin action. Based on this we further propose that this phenomenon may be exploited to provide a novel diagnostic application in predicting the response of the ovaries of a subject to ovarian stimulation, and further in determining the dose of gonadotropin to be administered to achieve ovarian stimulation.

In particular, we propose that monocytes, and related tissue-resident cells, specifically macrophages, sequester exogenous gonadotropins and thus regulate ligand availability at ovarian binding sites. This theory may explain reduced gonadotropin sensitivity in states of excessive macrophage activation, like obesity. We further propose and provide novel assays for estimating and predicting individual response to exogenous hormones.

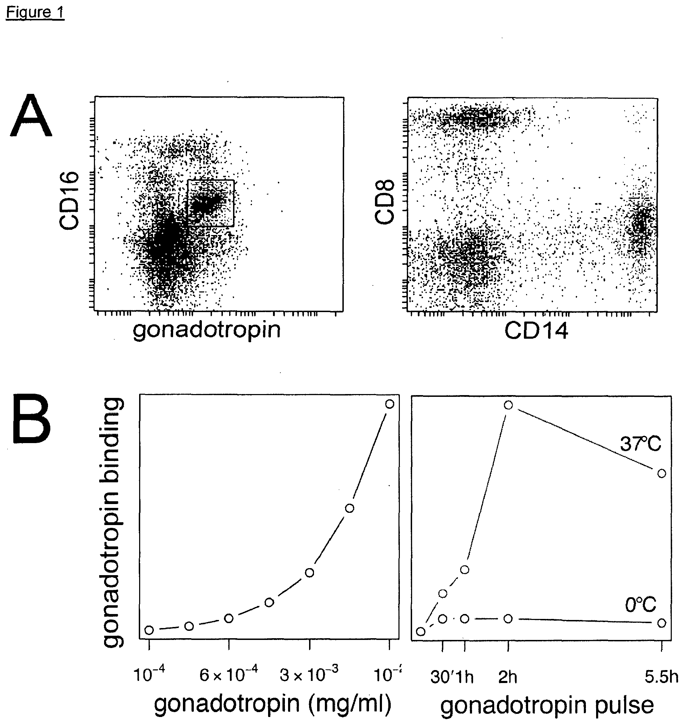

Our studies have shown that monocytes sequester gonadotropins *in vitro,* which predicts a reduced gonadotropin effect *in vivo.* In particular, while studying hormone action on ovarian cells, we have accidentally discovered that gonadotropins avidly bind to leukocytes *in vitro.* In particular, Example 1 shows by FACS analysis that blood-derived leukocytes incubated with fluorochrome conjugates of gonadotropins (FSH, LH, and hCG) bound to a sub-population of leukocytes bearing monocyte markers (See Fig 1A).

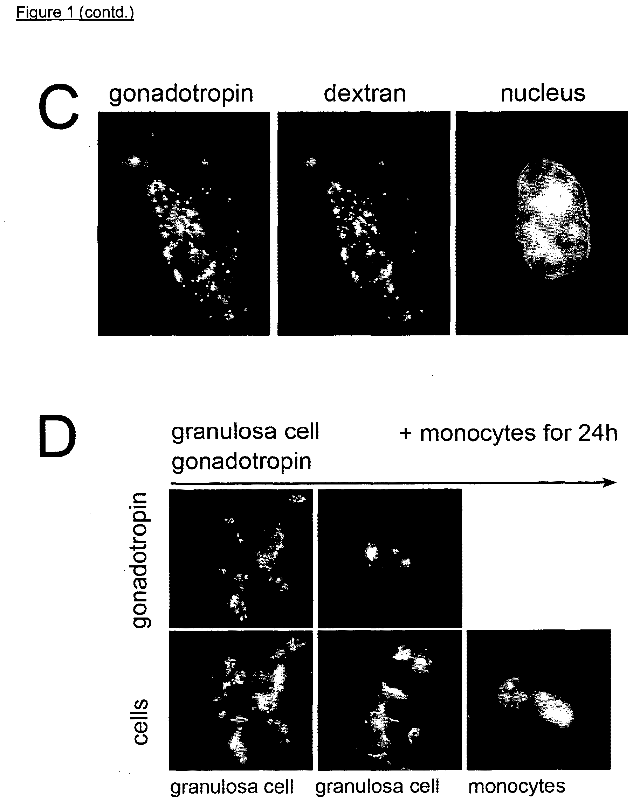

Further results showed that the molecules were internalized and suggested a macropinocytic mechanism of uptake (Fig 1 B). In order to assess the effect of gonadotropin internalization *in vitro,* monocytes and ovarian granulosa cells were co-cultured in the presence of fluorochrome-conjugated hCG. Labelled hCG was removed from granulosa cells and accumulated by differentially labelled monocytes (Fig 1 D).

These data thus showed that monocytes internalize gonadotropins by macropinocytosis using a mechanism that is distinct from binding to gonadotropin receptors. Internalised gonadotropins may be inaccessible to target ovarian cells, and hence internalisation of gonadotropins in this way may reduce the amount of administered (exogenous) gonadotropin which is available in a subject during ovarian stimulation. Thus, sequestration of gonadotropin by monocytes and related cells such as macrophages may constrain gonadotropin action. Accordingly, in a subject in whom such gonadotropin-sequestering cells are elevated or particularly active in sequestering the gonadotropin, a given dosage of gonadotropin may not be sufficient or may otherwise not be optimally effective in stimulating ovulation. Conversely, in a subject with a lesser amount of such cells or a lesser degree of sequestration, the same dose may be unnecessarily high.

Injected gonadotropin hormones are taken up in the blood stream and transported to the target organs, the gonads. Experiments using radioisotope-labeled gonadotropins in rats determined that gonadotropin is also being targeted to the kidney and the liver, and it was affirmed that these organs remove the hormone from the circulation. Notably, specific enrichment of the label in the leukocyte-rich spleen was not observed, indicating that monocytes, which only present in the blood stream, would not significantly contribute to the removal of gonadotropin.

We postulate that tissue-resident macrophages in the liver and kidney, which are not easily accessible for sampling, may share relevant features with monocytes, such as a cell surface receptor repertoire that determines hormone internalization, and therefore tests on easily accessible monocytes may predict the function of inaccessible liver and kidney cells.

Further experimental results reported in Example 2 below confirm that uptake of gonadotropins into monocytes *in vitro* correlates negatively with ovarian response to stimulation. In particular increased uptake of gonadotropin by monocytes or macrophages is associated with a requirement for increased gonadotropin dosage. Such results accordingly support our proposal that sequestration of gonadotropin by isolated monocytes or macrophages *in vitro* can predict a clinical response to gonadotropin during assisted reproduction treatment, and can be used to determine dosage of gonadotropin, particularly the most appropriate or optimal dosage for a given subject.

Accordingly, in one aspect the present invention provides a method for predicting the ovarian response of a subject to ovarian stimulation with a gonadotropin, said method comprising:

(i) contacting monocyte or macrophage cells from said subject *in vitro* with a gonadotropin; and
(ii) assessing uptake of said gonadotropin into said cells;

wherein uptake, particularly increased uptake, of gonadotropin into said cells is indicative of reduced ovarian stimulation.

In other words, the uptake of gonadotropin into the cells correlates negatively with ovarian stimulation. Thus, uptake of gonadotropin into monocyte or macrophage cells of a subject *in vitro* indicates that the subject is likely to demonstrate reduced ovarian stimulation on administration of gonadotropin to said subject, i.e. a reduced ovarian response to ovarian stimulation with gonadotropin.

Thus, the method of the invention above may include a further step of (iii) identifying said subject as requiring an increased (or higher) dosage of gonadotropin to stimulate an ovarian response, or as requiring (or being able to receive) a reduced (or lower) dosage of gonadotropin to stimulate an ovarian response. The dosage may be increased or reduced as compared to a standard dosage, e.g. as typically used in ovarian stimulation, or a reference dosage, or a dosage determined for the subject by other means, or example by reference to clinical indications e.g. conventional clinical indications in practice today.

This method of the invention may be used to determine the dosage of a gonadotropin to administer for ovarian stimulation of a subject. Generally, this will be in the context of assisted reproduction treatment.

In another aspect, the present invention accordingly provides a method for determining a dosage of a gonadotropin to be administered to a subject for ovarian stimulation of said subject, said method comprising :

(i) contacting monocyte or macrophage cells from said subject *in vitro* with a gonadotropin:
(ii) assessing uptake of said gonadotropin into said cells; and
(iii) determining said dosage from the degree of uptake.

In particular, in this method, uptake, particularly increased uptake, of gonadotropin into said cells is indicative of decreased availability of said gonadotropin to the ovary(ies) (more particularly to ovarian cells). Uptake of gonadotropin into monocytes or macrophages is hence indicative of a requirement for an increased dosage of gonadotropin.

Accordingly, in step (iii) of the method, the degree of uptake may be correlated to a dosage.

In a still further aspect, the present invention provides a kit which may be used in predicting the ovarian response of a subject to ovarian stimulation with a gonadotropin, said kit comprising:

(i) a gonadotropin; and at least one of
(ii) means for assessing uptake of said gonadotropin into monocyte or macrophage cells; or
(iii) means for isolating macrophage or monocyte cells from a subject; or
(iv) monocyte or macrophage test cells, and
(iv) means for determining the dosage from the degree of uptake, preferably a reference chart which correlates degree of uptake to dosage. This is discussed further below.

As will be apparent from the above, by correlating the degree of uptake of gonadotropin by monocytes or macrophages *in vitro* to the degree or level of ovarian response, an appropriate dosage of gonadotropin to be administered may be determined, to achieve ovarian stimulation in a subject. Thus the degree of uptake may be correlated to a dosage for ovarian stimulation. In particular, increased uptake may be indicative of a requirement for an increased dosage. Such a dosage, determined according to the methods of the invention, may be administered to a subject.

Accordingly, in a further aspect the present invention may inform a method of treatment in which a dosage of gonadotropin, determined according to the methods of the invention for determining a gonadotropin dosage, is administered to a subject (e.g. a patient). In particular, a dosage is administered to a subject in need thereof, more particularly a subject in need of ovarian stimulation. Advantageously, the dosage administered is effective in stimulating an ovarian response in said subject. An ovarian response is discussed further below, but may include stimulating the development of a follicle, which may include, but does not require, stimulating ovulation (the release of an ovum).

In particular the dosage may be determined from the degree of uptake, as indicated above.

Accordingly, the present invention includes a method for determining a dosage of a gonadotropin to be administered to a subject for ovarian stimulation of said subject and administering said dosage, said method comprising:

(i) contacting monocyte or macrophage cells from said subject *in vitro* with a gonadotropin;
(ii) assessing uptake of said gonadotropin into said cells;
(iii) determining said dosage from the degree of uptake; and
(iv) administering said dosage to said subject.

In particular, in step (iii) of the method, the degree of uptake may be correlated to a dosage.

Expressed in another way, the method may help inform a method of ovarian stimulation of a subject, said method comprising:

(a) determining a dosage of a gonadotropin to be administered for ovarian stimulation of said subject; and
(b) administering said dosage to said subject;

wherein said determining step (a) comprises:

(i) contacting monocyte or macrophage cells from said subject *in vitro* with a gonadotropin;
(ii) assessing uptake of said gonadotropin into said cells; and
(iii) determining said dosage from the degree of uptake.

In step (iii) of the method, the degree of uptake may be correlated to a dosage.

In a further aspect, the method described above may be adapted to provide a method of screening, or assay, for agents that modulate gonadotropin activity by determining the effect of said agents on uptake of gonadotropin by monocytes or macrophages *in vitro.* On the basis proposed above, agents which affect gonadotropin uptake by monocytic cells, may be expected to have an effect on gonadotropin activity, since they affect the amount of gonadotropin which is available (more particularly, available after administration to a subject).

Accordingly, the method of screening for an agent that modulates gonadotropin uptake by monoctye or macrophage cells, could include:

(i) contacting monocyte or macrophage cells *in vitro* with a gonadotropin before, after or simultaneously with a test agent
(ii) assessing uptake of said gonadotropin into said cells;
(iii) determining whether the test agents affects the uptake of said gonadotropin by said cells.

Thus, in such a method step (iii) may comprise comparing the uptake of gonadotropin by said cells in the presence or absence of the test agent. This comparison may be made in the course of the assay method. Alternatively, uptake in the presence of the test agent may be compared with a reference value, or predetermined measure of gonadotropin uptake by the test cells, for example as determined in the absence of the test agent. From the above, it will be understood that "in the presence" may mean that the test agent has been administered (contacted or added) to the cells before, during or after contact (or addition) of the test cells with gonadotropin, as long as the contact with the test agent is such that the test agent is able, or available, to have an effect on the test cells.

"Modulate" as used herein encompasses any effect on the level or degree or amount or rate of uptake. The agent may increase in decrease (reduce) the uptake (e.g. the amount of gonadotropin taken up, or the rate of uptake). A "test agent" is an agent whose effect on gonadotropin activity is to be determined.

The test cell may be any monocyte or macrophage cell. For example, monocytes or macrophages isolated from a human or non-human animal subject may be used. Freshly isolated or stored or treated cells may be used. Alternatively monocytic cell lines may be used, e.g. a cell line derived from any monocyte or macrophage cell. For example myelomonocytic leukaemia cell lines may be used, and various such cell lines are mentioned in Example 4.

In step (iii) of the method, the degree of uptake indicates whether the agent has an effect, e.g. a positive, negative or no effect, on gonadotropin uptake. As discussed above, uptake and sequestration of gonadotropin by monocytes and macrophages may constrain gonadotropin action (by reducing its availability). Accordingly, the effect of an agent(s) on gonadotropin uptake by said cells may be correlated to an effect on activity. Thus the method of this aspect of the invention may also be viewed as method for determining the effect of an agent on gonadotropin activity, or for determining whether an agent modulates gonadotropin activity, or screening for an agent that modulates gonadotropin activity.

Kits may further be provided for performing such a screening or determination method. Suitable kits are defined above and may optionally include monocyte or macrophage test cells, e.g. cells of a cell line, such as a monocytic cell line, e.g. a myelomonocytic leukaemia cell line.

Thus kits as defined above, may also be used in a method (assay) to screen for an agent(s) that modulates gonadotropin activity by determining the effect of said agent(s) on the uptake of gonadotropin by monocytes or macrophages *in vitro*.

Accordingly, the kit may optionally comprise a test agent. Alternatively, the test agent may be provided by the end user. The kit may additionally or alternatively further comprise a means for comparing the level uptake of gonadotropin in the presence of said test agent with a reference value or a predetermined measure of gonadotropin uptake by the cells and correlating the effect of an agent(s) on gonadotropin uptake by said monocytes or macrophages to an effect on activity of the gonadotropin. Such means may be, for example, a reference chart.

Gonadotropins are large glycoprotein hormones of the pituitary gland consisting of a common $\alpha$ subunit and a hormone-specific $\beta$ subunit. The latter is glycosylated at various sites, and the level and pattern of glycosylation affects specificity, receptor binding, and pharmacokinetics of the hormone. Indeed, the structure of linked oligosaccharide chains appears to be related to serum half-life, and explains the largely differing kinetics of FSH, LH and hCG. As used herein the term "gonadotropin" includes any such hormone, whether natural or synthetic, in wild-type or native form, or modified, as long as gonadotropin activity is retained (that is the hormone activity of the gonadotropin, and in particular the effect of the molecule in stimulation or inducing development of a follicle, or in causing or contributing to ovulation). Thus, the term includes in particular follicle-stimulating hormone (FSH), luteinizing hormone (LH) and chorionic gonadotropin (CG) or a mixture of one or more of these. For example, human menopausal gonadotropin (HMG) is a preparation derived from the urine of post-menopausal women. HMG contains a mixture of gonadotropins FSH and LH. Various forms of CG are known, e.g. hCG and eCG. The gonadotropin polypeptides may be sequence-modified, e.g. by deletion, insertion, addition or substitution of one or more amino acids. Various recombinant gonadotropin variants are known. The gonadotropin may also be modified by chemical derivatisation, e.g. modification or addition of one or more glycosyl groups, or addition of or substitution of one or more groups or chemical moieties on the amino acid residues.

Pharmacokinetic studies on radiolabelled human gonadotropins in rats indicate that after absorption, the hormone is rapidly distributed to the periphery, including the ovaries, and approximately 80% is excreted by the kidneys. In addition, gonadotropins are sequestered in the liver by a specific lectin of the reticuloendothelial cells. Indeed, receptor-mediated uptake in the liver was shown to be a main regulation of serum glycoprotein levels, including LH.

We postulate that monocytes and macrophages may possess a similar receptor repertoire to liver reticuloendothelial cells, and gonadotropin sequestration in monocytes or macrophages may correlate with that in the liver, which in turn largely determines the level of free bioactive hormone.

Monocytes and macrophages are phagocytic leukocytes. Monocytes are a type of white blood cell and are part of the immune system of all vertebrates, playing multiple roles in immune function. Blood monocytes migrate into the tissues of the body and there they may differentiate into macrophages or dendritic cells. According to the present invention, any monocyte or macrophage cell, or a related tissue-resident cell, may be used in the methods. Monocytes and macrophages may be recognised or isolated based on characteristics and procedures well known in the art. For example, monocyte markers may be used to define or isolate an appropriate population of cells, e.g. monocyte and macrophage cells.

Most notably, the cells may be identified on the basis of the expression of the marker CD14. Thus, in alternative definitions of the methods (and kits) of the present invention, rather than being referred to as monocytes and macrophages, the cells to be used may be defined as leukocytes expressing CD14 (i.e. CD14$^+$ leukocytes), particularly peripheral leukocytes expressing CD14, and more particularly as CD14$^{high}$ leukocytes.

More particularly, the cells may be defined as a subset of leukocytes, or peripheral leukocytes, expressing CD14 which are negative for lineage markers of T-cells, B-cells, and granulocytes. Even more particularly, the cells are a defined subset of peripheral leukocytes, which express the monocyte markers CD14$^{high}$, CD16$^{low}$, CD11c, but which are negative for lineage markers of T-cells, B-cells, and granulocytes.

For use in the methods of the invention, the cells may be obtained from any sample of the subject which may contain the cells. Conveniently the sample will be blood or a white blood cell-containing sample derived from blood. However, the cells may be obtained from other parts or tissues of the body, for example the spleen. The sample may thus be a tissue biopsy sample. The tissue may be from any part of the body where monocytes or macrophages may be found. As noted above macrophages may be resident in a number of different tissues.

The subject may be any human or non-human female animal subject, in particular a female subject of reproductive age, and/or post-pubertal (e.g. an adult female). Whilst human subjects are preferred the methods of the invention may also find utility in the fertility treatment of other animals such as livestock animals, pets, race horses etc. The methods of the invention may find application in animal breeding.

The term "predicting" as used herein relates to any method of determining or estimating the expected ovarian response of the subject. The "ovarian response" refers to the endocrine and follicular reaction of the ovaries to a stimulus. In the case of the present invention this will generally be the growth and development of one or more follicles in the ovary. This may culminate in ovulation (i.e. the release of an egg by an ovary), but, as is well understood in the field of assisted reproduction, ovulation need not necessarily occur, and the ovarian response may be the development of one or more follicles, preferably multiple (e.g. two or more) follicles. Particularly the follicles will be suitable for harvest for use in IVF procedures.

The stimulus will generally be an exogenous gonadotropin, i.e. the administration of a gonadotropin to the subject. "Ovarian stimulation" refers to the administration of exogenous gonadotropin to a subject. This will generally be with the aim or purpose of stimulating an ovarian response.

In the context of determining the dosage of the gonadotropin, the term "determining" refers generally to any means of assessing or estimating or calculating the amount of the dosage required. This is discussed in more detail below.

"Contacting" the cells with the gonadotropin includes any means of applying or "administering" the gonadotropin to the cells, such that the gonadotropin is accessible to the cells, and in particular accessible for uptake by the cells. Conveniently the cells may be incubated with the gonadotropin. "Uptake" refers generally to the entry of the gonadotropin into the interior of the cells, i.e. transport across the cell membrane into the cell, howsoever achieved. Uptake may thus alternatively be referred to as "internalisation".

Uptake may be "assessed" by any convenient means and accordingly "assessing" refers generally to any means of determining, estimating or measuring uptake, e.g. the amount of gonadotropin which has been taken up or internalised by the cell. Different ways of assessing uptake are described further below.

The "degree" of uptake may accordingly be viewed as any indicator of the amount of gonadotropin taken up by the cell.

To allow uptake of gonadotropin to be assessed, the gonadotropin which is contacted with the cells *in vitro* is advantageously labelled, to allow detection of the gonadotropin. By "labelled" it is meant that the gonadotropin is provided with or carries any label which may be detected, either directly or indirectly. The label may be directly signal-giving or indirectly signal-giving. For example a directly signal-giving label directly gives rise to a signal which may be detected directly, for example spectrophotometrically or visually. Thus the label be a coloured or fluorescent or radioactive label (e.g. a dye or radioisotope) or it may a particulate label which may be detected by visual inspection e.g. by microscopy, e.g. colloidal particles (e.g. colloidal gold) or any label which can be detected or distinguished by any means e.g. by size or density etc. An indirectly signal-giving label may give rise to a detectable signal upon further treatment of reaction, e.g. contact with a further substance e.g. an enzyme. Thus a signal may be developed from an indirectly signal-giving label by a subsequent reaction, e.g. an enzymatic or chemical reaction, or by binding to a further substance. An indirectly signal-giving label may be chromogenic or may give rise to a fluorescent signal upon further reaction or binding. For example, such a label may include biotin which may subsequently be detected by means of reaction which involves binding to streptavidin.

To practice the methods of the invention, the cells to be contacted (i.e. the monocytes or macrophages, or alternatively the CD14+ leukocytes etc) may be isolated from the subject before contact with the gonadotropin. Alternatively, cells from the subject, e.g. cells from a sample of the subject, e.g. a blood sample may be contacted with the gonadotropin before the test cells are isolated. Thus the step of contacting cells with the gonadotropin may take place prior to, after, or simultaneously or contemporaneously with a step of isolating or identifying the desired test cell population (e.g. macrophages and/or monocytes) from the subject or from a sample from the subject.

For example leukocytes may be isolated from a blood sample using known techniques (e.g. by density gradient centrifugation) and may then be contacted with gonadotropin, e.g. labelled gonadotropin. Such leukocytes may for example be a leukocyte fraction such as peripheral blood mononuclear cells (PBMC). Monocytes or macrophages may then be isolated or detected, for determination of gonadotropin uptake. Conveniently this isolation or detection may be achieved by the use of monocyte markers such as CD14. For example reagents may be used which bind to the marker, e.g. any affinity binding molecule or ligand (e.g. an antibody or antibody fragment etc, or an aptamer or any ligand identified by a screening or selection procedure as being capable of binding to the marker). Antibodies, and their fragments, including both monoclonal and polyclonal antibodies and various derivatives such as chimeric antibodies, single chain antibodies, CDR-grafted antibodies, etc., are well known and widely described in the art. Such binding molecules or ligands may be labelled to allow their detection. Thus, detection of a monocyte marker may be used to identify the test cells in which uptake is to be assessed. The use of flow cytometry procedures and in particular FACS may advantageously allow the required leukocyte subset to be identified (e.g. by the use of a detectable reagent which binds to a monocyte marker such as CD14) and for uptake of the gonadotropin to be assessed, e.g. by detecting the gonadotropin label.

Alternatively, the appropriate or desired test cells may first be isolated from the subject or sample on the basis of the markers they express for example by binding to a binding molecule, as described above (e.g. an antibody or antibody fragment, etc.), which may bind to a marker on the cell surface, which binding molecule is immobilized, or may be immobilized, on a solid support. Any convenient solid support may be used, for example particles (e.g. magnetic particles) or any plate, well, strip, slide or vessel etc.

Various different procedures for isolating and/or identifying monocytes and/or macrophages and for contacting them with gonadotropin may be used.

In one such procedure, as mentioned above, leukocytes are isolated from blood. PBMC are incubated with labelled gonadotropin, followed by binding to a labelled molecule capable of binding to a cell surface expressed monocyte marker (e.g. CD14). Such a binding molecule may typically be an antibody, particularly a monoclonal antibody or a fragment thereof, e.g. against CD14. Such molecules may be labelled by direct conjugation to a reporter molecule such as a fluorophore. For example, to detect CD14-expressing cells, monoclonal antibodies raised against CD14 may be used. These antibodies may be directly conjugated with a fluorophore, e.g. PerCP (peridinin-chlorophyll complex protein), and termed anti-CD14-PerCP. Anti-CD14-PerCP then binds to CD14 on the cell surface and decorates CD14-expressing cells. Using PerCP is advantageous because it allows simultaneous observation of FITC and PerCP signals using different fluorescence detection channels. FACScan analysis is then used to determine uptake of labelled gonadotropin into monocyte marker-positive (e.g. CD14 positive) cells.

Other representative procedures include:

1. Radioiodinated gonadotropin

Human gonadotropin is conjugated to a radioactive isotope, e.g. $I^{125}$ isotope. Isolated peripheral monocytes are incubated with [$I^{125}$]-gonadotropin on ice or at 37°C. Cells are harvested onto an absorbent paper and cell-incorporated radioactivity is measured. Specific uptake is calculated by subtracting uptake on ice.

2. Beads

Whole blood or isolated leukocytes are incubated with labelled (e.g. FITC-labelled) gonadotropin on ice or at 37°C. Monocytes are then selected with paramagnetic immunobeads, or any other suitable solid support, conjugated with an antibody (etc.) or other molecule capable of binding to CD14 or other monocyte-specific marker. The selected cell population is assessed to determine uptake of gonadotropin, e.g. the amount of internalised label. For example cells may be observed under an epifluorescent microscope to estimate the amount of internalized gonadotropin.

3. Fluorometer-based assay

Whole blood or isolated leukocytes are incubated with labelled gonadotropin (e.g. FITC-gonadotropin) on ice or at 37°C. 96-well plates are coated with anti-CD14 or specific antibody raised against other monocyte markers (or any other suitable binding molecule against a monocyte marker). Monocytes are captured on the coated surface, and the amount of internalized gonadotropin is measured by a microplate reader capable of detecting the label, e.g. fitted with a fluorescein-specific filter.

4. Multiwell assay for spectrophotometer

Gonadotropin may be conjugated to biotin. Whole blood may be incubated with labelled gonadotropin, followed by cell separation with CD14 antibody-conjugated paramagnetic beads. Isolated cells may be lysed in streptavidin-coated wells followed by detection of released biotin-conjugated gonadotropin with standard ELISA reagents.

It will seen therefore that a range of different procedures may be used to carry out the steps of the method, and that the cells may be contacted in different ways, and uptake may be assessed in different ways. Thus, uptake may be assessed by detecting internalised gonadotropin in the cells (e.g. by visualisation microscopically, or by detecting the label of internalised gonadotropin). Alternatively, the internalized gonadotropin may be released by lysing the cells and detecting (e.g. determining the amount of) the released gonadotropin. Thus FACS, immunoassay (e.g. ELISA), microscopic, radioisotope detection, fluorometer- or other spectrophotometer-based assays may be used.

In the methods of the invention the gonadotropin which is contacted with the cells will generally be the same gonadotropin which is intended for use in ovarian stimulation of the subject. However, it need not necessarily be so, and for example a different variant of the gonadotropin may be used for the test to that which will be administered to the subject.

The methods of the invention may allow the ovarian response of a given subject to a given gonadotropin dose to be predicted. Thus it may be determined in advance whether that dose is likely to be effective.

As noted above, in another aspect of the invention, the methods may involve determining the dosage of gonadotropin to be used for ovarian stimulation in a subject. As described above, dosage is related to the degree, or amount, of uptake of gonadotropin into monocytes or macrophages of the subject. Thus, the greater the degree of uptake, the lesser the degree of ovarian stimulation expected by the gonadotropin (since less of it will be available for ovarian stimulation of the subject, as it will taken up by the monocytes or macrophages in the subject). Accordingly, increased uptake is indicative of decreased stimulation. In turn, this indicates that an increased dosage is required for that subject.

The method of the invention may inform the administration of a gonadotropin. The administration may be by any suitable method known in the medicinal arts, including for example parenteral (e.g. intramuscular, subcutaneous, intraperitoneal or intravenous), oral, rectal, subcutaneous, percutaneous, transmucosal, buccal, nasal, vaginal, transdermal or topical administration. Preferred forms of administration are the patenteral and subcutaneous routes.

The gonadotropin may be formulated for administration according to well known in the art. As such, the gonadotropin may comprise any known carrier, diluent or excipient. For example, formulations which are suitable for parenteral administration conveniently comprise sterile aqueous solutions and/or suspensions of pharmaceutically active ingredients preferably made isotonic with the blood of the recipient, generally using sodium chloride, glycerin, glucose, mannitol, sorbitol and the like. Therefore, a gonadotropin composition may comprise a pharmaceutically acceptable carrier, such as aqueous solution, non toxic excipients, including salts, sugars, amino acids, surfactants, preservatives, stabilisers, isotonicity agents, buffers and the like. Examples of suitable aqueous and non-aqueous pharmaceutical carriers, diluents, solvents or vehicles include water, ethanol, polyols (such as glycerol, propylene glycol, polyethylene glycol, and the like), carboxymethylcellulose and suitable mixtures thereof, vegetable oils (such as castor oil), and injectable organic esters such as ethyl oleate. The gonadotropin composition may also comprise preservatives, wetting agents, emulsifying agents, and dispersing agents. Antibacterial and antifungal agents can be included to prevent growth of microbes and includes, for example, paraben, chlorobutanol, phenol, sorbic acid, and the like. Furthermore, it may be desirable to include isotonic agents such as sugars, sodium chloride, and the like.

Gonadotropins may be obtained from any source known to the skilled person. As noted above, the term gonadotropin includes in particular FSH, LH and CG or a mixture of one or more of these. The term also includes recombinant versions of the compounds. Recombinant FSH products include, for example Puregon (Organon), Gonal F and Follistim. Methods of expressing and purifying recombinant FSH (e.g. from CHO or human cell lines) are well known in the art. FSH preparations also include hMG (human menopausal gonadoropins; a mixture of LH and FSH). Products containing FSH and LH include Pergonal™, Repronex™, Humegon™, and Menopur™, and those containing mostly FSH include Fertinex™, Metrodin™ and Bravelle™. hCG products include Pregnyl™, Novarel™, Profasi™ and Ovidrel™.

Administration of the gonadotropin may be in a single dose, such as a single dose taken at regular intervals, or may be administered as divided doses to be taken for example during the course of a day (e.g. 1 to 4 times a day). Administration may carried out on a single day or over a number of consecutive or non-consecutive days (e.g. 1 to 21 days or more). The precise dosage of the gonadotropin to be administered, the number of doses and the length of the course of treatment will depend on a subject to whom the gonadotropin is to be administered and may be readily determined by the medical practitioner. As explained herein, this determination may achieved using the methods of the present invention.

The daily dosage of gonadotropin may be in the range of 50 - 600 IU, preferably 50 - 400 IU, 50 - 300 IU, 75 - 300 IU, 100 - 300 IU, 100 - 275 IU, 100 - 250 IU, 110 - 250 IU, 110 - 230 IU. A representative daily dosage range of gonadotropin may be 115 - 225 IU. A typical daily dosage is 150 IU.

By way of example, for a "standard" (i.e. "average") patient, under 35 years of age, with a body mass index 18-30 kg/m$^2$, without co-morbidity, normal gynaecological findings, and no prior treatments, this patient receive a 150 IU daily starting dose of gonadotropin(s). 150 IU may therefore be regarded as a "standard" daily dose. If the patient is found to have increased ovarian follicle count (e.g. >10 follicles, 2-8 mm diameter) as determined e.g. by ultrasound scan or high serum anti-Müllerian hormone levels (e.g. >20 pmol/l), indicating that she may respond excessively to hormones, the starting dose may be set to around 115 IU. If, for example, the patient was overweight with few follicles, the dose may be set to 200-225 IU per day.

It should be noted that these values only apply to therapy-naive patients who have never before undergone IVF. Once a stimulation protocol has been completed, it is possible to more accurately estimate the correct dose for the next stimulation.

The step of determining the dosage accordingly involves correlating, or associating, or relating the degree of uptake to the dose required for ovarian stimulation of that subject. Advantageously, an optimum dose may be determined, that is a dose which achieves a sufficient ovarian response, but which minimizes the risk of undesirable side effects. As noted above, these may include ovarian hyperstimulation syndrome. Undesired side effects may also include deleterious effects on the embryo, e.g. genetic effects.

In order to allow such correlations of uptake to dose, reference charts or graphs etc (e.g. a nomogram) may be prepared based on results of tests with different subjects and at different doses (e.g. of different gonadotropins) which link or associate a particular uptake to a particular dosage to be administered. Techniques and methods for preparing such reference charts are known in the art. Accordingly, the step of determining the dosage may comprise the step of comparing the degree of uptake to a reference chart to determine the dose to be administered. The reference chart may be previously determined, or may be a "standard" reference chart.

Indeed the degree of uptake may be combined with other parameters determined for the subject, either to predict ovarian response or to determine dosage. Such other parameters may include other relevant clinical or biochemical variables such as age, ovarian volume, serum FSH or serum AMH (anti-Müllerian hormone) or other known or recognised predictors or indicators of ovarian reserve. Accordingly, the reference chart (e.g. nomogram) which is constructed may relate to a combination of features, of which uptake of gonadotropin into monocyte or macrophage cells is one. This may allow a more precisely tailored dosage to be determined, that is a dosage which is individually tailored to the subject.

The methods of the invention thus have a number of advantages and applications. For example by predicting ovarian response and determining a dosage appropriate for a particular subject erroneously high or low gonadotropin doses during ovarian stimulation for IVF may be avoided. Using the methods of the invention, a reference or dosage chart (e.g. a nomogram) may be established and used to determine correct gonadotropin doses in order to reduce the risk of treatment side effects and improve efficacy (see e.g. Example 3). Undesirable side effects and consequences may include:

(i) Ovarian hyperstimulation syndrome (OHSS). OHSS is a serious, potentially fatal complication of IVF due to increased vascular permeability that may cause intractable ascites and multi-organ failure. Its incidence is 1-23% depending on severity and it is the most common cause of IVF-related hospitalization.

(ii) Adnexal torsion. Enlarged ovaries have an increased tendency for torsion compressing the vascular pedicle and causing acute abdomen. It is a rare complication (3% of gynaecological surgeries) with increased risk after IVF.

(iii) Inferior treatment outcome due to low oocyte count or gamete aneuploidy. Approximately 10-20% of initiated IVF treatments are not completed because no embryo was available for transfer. This risk is directly related to oocyte count, as only 25% of retrieved oocytes develop into viable embryo of fair quality. Furthermore, erroneously high gonadotropin dosage is estimated to increase the incidence of chromosomally abnormal embryos by 50%.

(iv) Treatment cancellation due to risk of OHSS or multiple pregnancy. Treatments with gonadotropins are often abandoned due to exaggerated ovarian response, in order to avoid the risk of OHSS or, in case of insemination or ovulation induction, the risk of multiple ovulation and high order multiple pregnancy.

Other applications include predicting ovarian response to a given gonadotropin dose.

Using the methods of the invention it may be possible to estimate ovarian response to gonadotropin stimulation before treatment initiation. This prognostic information is highly relevant for clinical routine, since the choice to offer or deny treatment for a given couple is often based on anticipated poor response to stimulation (currently assessed by age, response during prior treatments, serum levels of FSH or AMH).

The methods may also enable improved treatment choice. Thus, the proposed method can be used to identify patients with altered gonadotropin pharmacokinetics who may benefit from alternative treatment regimens (e.g. more prolonged or shorter down-regulation with GnRH agonist, treatment with GnRH antagonist, step-up or step-down regimens) or alternative drugs (e.g. aromatase inhibitors).

The method of the invention may extend the utility of new devices that allow accurate gonadotropin dosage. Some gonadotropins are now available in multiple dosage devices (pen), which allow dose adjustment with 8.33 units. These possibilities are seldom exploited in the clinical practice, however, and patients routinely receive prescription for 75 IU, 150 IU, or other multiples of 75 IU, the traditional dose in a single gonadotropin ampoule.

The methods of the invention may find application in dose titration. Thus, the methods may help in devising charts (e.g. nomograms) to calculate the mono-ovulatory gonadotropin dose. In ovulation induction treatment, gonadotropins are given to induce development of a single ovarian follicle and multiple ovulation is undesired due to risk of multiple pregnancy. Finding the right dose for mono-ovulation is challenging, and may require several titration cycles.

As noted above, predictive charts and devices may be established using the methods of the invention. The method of the invention, together with other relevant clinical and biochemical variables (age, ovarian volume, serum FSH, serum AMH) may allow development of prediction models and calculation of individually tailored gonadotropin doses. This would be of particular benefit for women of advanced reproductive age (35 years of age and above) who may experience highly variable response during stimulation.

Other uses may lie in cell-based high throughput screening tests for modulators of gonadotropin pharmacokinetics. The method may be adapted to a high throughput screening (HTS) platform to examine large libraries of chemical substances and identify compounds that may regulate macropinocytosis by monocytes with possible modulatory effect of hormone pharmacokinetics.

As noted above, the invention also includes kits for use (or when used) in the methods of the invention.

This aspect of the invention provides a kit for use in predicting the ovarian response of a subject to ovarian stimulation with a gonadotropin, said kit comprising:

(i) a gonadotropin; and at least one of
(ii) means for assessing uptake of said gonadotropin into monocyte or macrophage cells; or
(iii) means for isolating macrophage or monocyte cells from a subject.

The kit may further optionally comprise (iv) means for determining the dosage from the degree of uptake. Such means may be a reference chart, e.g. nomogram, as discussed above.

Advantageously, the gonadotropin in the kit is labelled.

The means for assessing uptake may include means for detecting the label of the labelled gonadotropin. Alternatively, the means may include further components of the signal giving reaction used to determine uptake. As described above, this may include means for lysing the cells and for detecting the released gonadotropin. Such detecting means may include reagents for assay e.g. immunoassay, of the gonadotropin.

Means for isolating macrophage or monocyte cells may include binding molecules capable of binding to cell surface markers on monocytes or macrophages. e.g. a binding molecule (i.e. ligand) capable of binding to CD14, more particularly a binding molecule capable of binding specifically to such a marker. Such a binding molecule may be labelled for detection (directly or indirectly) or may be immobilised or immobilisable on a solid support.

The invention will now be described in more detail in the Examples below with reference to the following drawings in which:

Figure 1 shows uptake and sequestration of fluorescein-conjugated gonadotropin by peripheral blood monocytes *in vitro*;

Figure 2 shows uptake of FITC-labelled HMG by CD14[+] monocytes *in vitro* correlates with ovarian response to gonadotropin stimulation *in vivo* among 73 women undergoing ovarian stimulation for IVF.

Figure 3 shows a nomogram to estimate ovarian response during hormone stimulation for IVF based on serum concentration of AMH, body mass index, and uptake of FITC-labeled HMG *in vitro*.

Figure 4 shows percent inhibition of gonadotropin uptake by HEL, ML2, THP1, and U973 cells *in vitro* pre-incubated on ice or exposed to cytochalasin D (CCD), 5-N,N-dimethyl-amiloride (DMA), $NaN_3$, or wortmannin (WORT).

Examples

Example 1

This study shows that monocytes sequester gonadotropins *in vitro,* which predicts a reduced gonadotropin effect *in vivo*. In particular, while studying hormone action on ovarian cells, we have accidentally discovered that gonadotropins avidly bind to leukocytes *in vitro*.

Materials and Methods

Patients

Blood sample were taken before prior to treatment of patients undergoing assisted reproduction. For practical reasons, the preliminary data were based on samples taken at the end of treatment.

Monocyte isolation and uptake of FITC-conjugated FSH

Blood-derived monocytes are enriched by density gradient centrifugation. Cells are split in two and incubated for 2 hours in the presence of FITC-conjugated FSH, at 37°C or on ice. Cells are labelled with fluorochrome-conjugated monocyte marker (e.g. anti-CD14-PerCP antibody, as described above) and analyzed with multi-colour flow cytometry (FACS).

Internalization of FSH is estimated as mean fluorescence intensity (MFI) of the cell population expressing monocyte markers. Unspecific binding (background) is estimated as MFI for cells incubated on ice

We incubated blood-derived leukocytes with fluorochrome conjugates of human recombinant and urine-derived gonadotropins (FSH, LH, and hCG). Cells were analyzed with flow cytometry (FACS) with simultaneous detection of multiple cell lineage markers. The results are shown in Figure 1.

All gonadotropins were found to bind to a defined subset of peripheral leukocytes, which expressed the monocyte markers CD14$^{high}$, CD16$^{low}$, CD11c, but were negative for lineage markers of T-cells, B-cells, and granulocytes (Fig 1A). Cell-bound gonadotropins could not be quenched with cell-impermeable trypan blue dye suggesting that the molecules were internalized. Gonadotropin binding could not be saturated with ligand and was inhibited by low temperature or cytochalasin, suggesting a macropinocytic mechanism of uptake (Fig 1 B). Internalized gonadotropins co-localized with internalized dextran, indicating lysosomatic targeting (Fig 1C). In order to assess the effect of gonadotropin internalization *in vitro*, monocytes and ovarian granulosa cells were co-cultured in the presence of fluorochrome-conjugated hCG. Labelled hCG was removed from granulosa cells and accumulated by differentially labelled monocytes (Fig 1 D).

From these data we concluded that monocytes internalize gonadotropins by macropinocytosis using a mechanism that is distinct from binding to gonadotropin receptors. Digested gonadotropins may be inaccessible to target ovarian cells, which may have interesting physiological applications-indeed, gonadotropin sequestration by the monocyte-macrophage system may be a significant regulatory pathway of ovarian function that constrains gonadotropin action.

Example 2

In order to explore the clinical relevance of gonadotropin internalization by monocytes *in vitro*, we isolated leukocytes from 73 women undergoing assisted reproduction treatment at the time of oocyte recovery.

Materials and Methods

Patients

Blood sample were taken before prior to treatment of patients undergoing assisted reproduction. For practical reasons, the preliminary data were based on samples taken at the end of treatment.

Monocyte isolation and uptake of FITC-conjugated FSH

Blood-derived monocytes are enriched by density gradient centrifugation. Cells are split in two and incubated for 2 hours in the presence of FITC-conjugated FSH, at 37°C or on ice. Cells are labelled with fluorochrome-conjugated monocyte marker (e.g. anti-CD14-PerCP antibody) and analyzed with multi-colour flow cytometry (FACS).

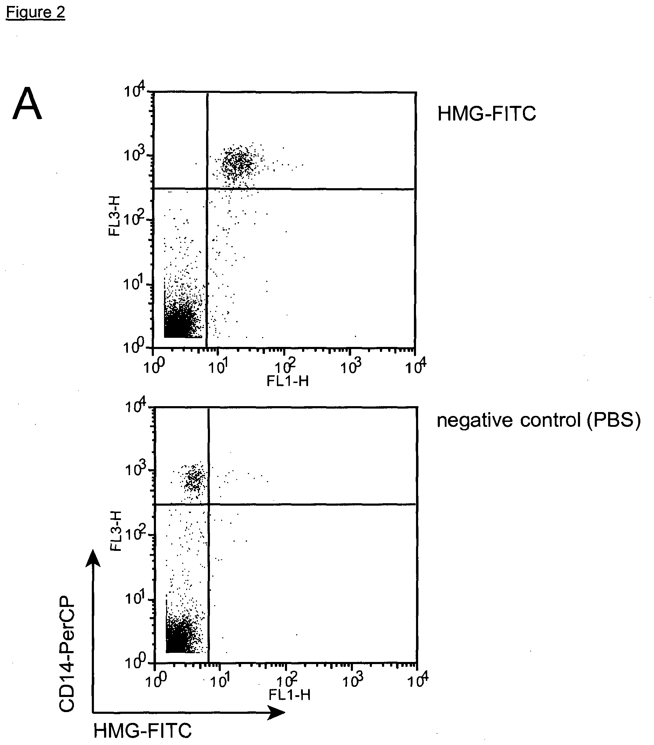

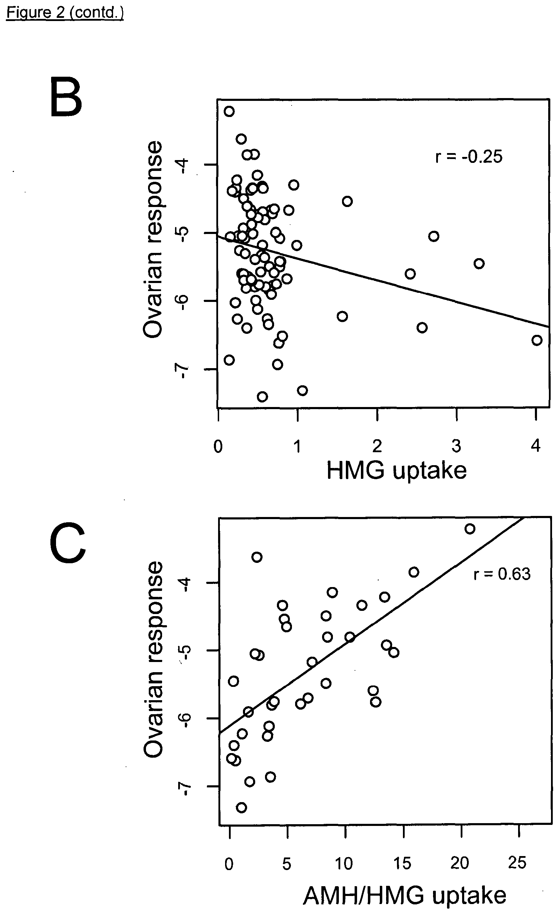

Internalization of FSH is estimated as mean fluorescence intensity (MFI) of the cell population expressing monocyte markers. Unspecific binding (background) is estimated as MFI for cells incubated on ice (see Figure 2, panel A).

Peripheral blood mononuclear cells were incubated with FITC-HMG for 1.5 hrs at 37 °C, followed by labelling with CD14-PerCP HMG-specific median fluorescence intensity of CD14$^+$ cells was determined using a FACScan instrument, with appropriate compensation settings for multi-colour observations (Fig 2A). Uptake of FITC-dextran, which enters the cells by obligate receptor-mediated endocytosis, was also measured as internal positive control. HMG uptake was expressed as ratio of median HMG-specific and dextran-specific fluorescent intensity.

HMG uptake was negatively correlated with ovarian response to stimulation, either expressed as oocyte count or response index, i.e. ratio of oocyte count and total gonadotropin dose (r = -0.25, P = 0.03, n = 73; Fig 2B). As expected, in a subset of women where AMH was measured, response to stimulation was positively correlated with serum AMH concentration (r = 0.44, P = 0.006, n = 37). An index derived as ratio of serum AMH concentration and HMG uptake showed superior association with ovarian response (r = -0.63, P < 0.0001, n = 37; Fig 2C).

Collectively, the findings indicate that an index comprising measures of ovarian reserve (AMH concentration) and gonadotropin metabolism (HMG uptake) may predict response to ovarian stimulation during IVF treatment.

Example 3

In order to construct a nomogram to predict response during ovarian stimulation from clinical and biochemical data obtained before treatment, we prospectively examined 56 women who were scheduled to undergo IVF.

Materials and methods

Patients were examined during their first outpatient visit. Height, weight, and body mass index (kg/m$^2$) were recorded. Serum concentration of anti-Müllerian hormone (AMH) was routinely measured during infertility work-up and was available at the outpatient visit.

A single blood sample was taken and was immediately processed by density gradient centrifugation to isolate monocytes, incubation with FITC-conjugated gonadotropin (HMG) or vehicle (PBS) at 37 °C, labelling with fluorochrome-conjugated monocyte marker (e.g. anti-CD14-PerCP antibody), and multi-colour flow cytometry (FACS). FITC-specific median fluorescence intensity (MFI) was assessed in the monocyte gate and HMG uptake was derived with the formula:

$$HMGuptake = \log \frac{MFI_{FITC-HMG}}{MFI_{PBS}}$$

Subsequently, 32 of the tested patients underwent ovarian stimulation with FSH, oocyte retrieval, and IVF according to standard procedures. All women received pre-treatment with gonadotropin-releasing hormone agonist from the midluteal phase of the preceding menstrual cycle. Women with anticipated normal response, i.e. under 35 years of age, body mass index of 18 - 30 kg/m$^2$, and no co-morbidities, typically received ovarian stimulation with 150 IU daily injections of FSH for 8 days, when the dose was adjusted according to response assessed by ultrasound scan and serum estradiol concentration. Women suspected of a reduced response, e.g. above 35 years of age, were given 200 - 225 IU as increased initial daily dose. Women anticipated to have an increased response, e.g. because of multiple ovarian follicles, were given a reduced initial daily dose of 50 - 115 IU FSH. Ovarian response index (ORI) was defined as:

$$ORI = \log \frac{(No.\,of\,retreived\,oocytes)}{Total\,FSH\,dose\,(IU)}$$

Multiple regression analysis was used to predict ORI using AMH and BMI, or AMH, BMI, and HMGuptake as independent variables. These regression models were compared with analysis of variance. The model containing the HMGuptake term was superior to the regression model with AMH and BMI (P = 0.03). Increased AMH was associated with higher ORI (b = 0.004, P = 0.07), whereas increased BMI (b = - 0.03, P = 0.006) and HMGuptake (b = -0.20, P = 0.04) were associated with decreased ORI.

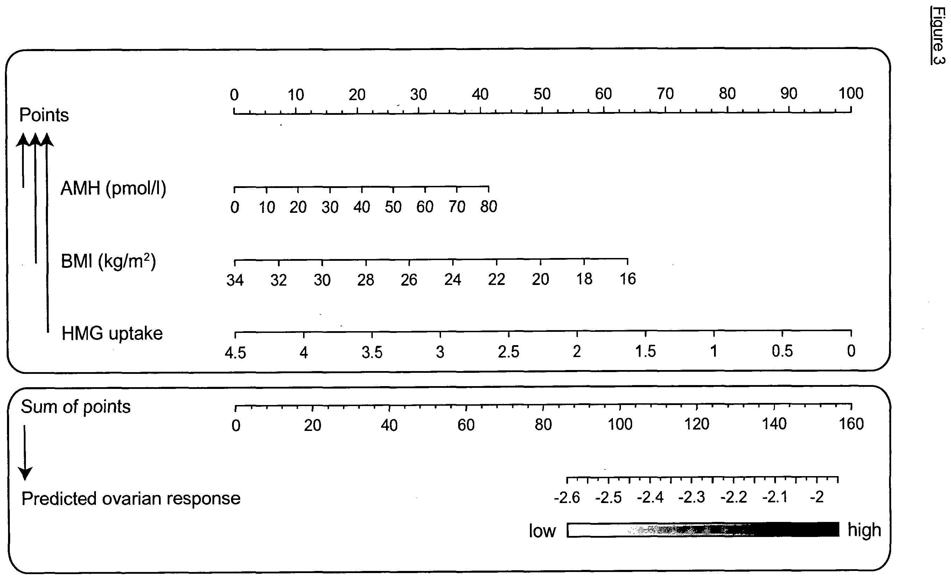

Based on this regression model, a nomogram was constructed where measured values for AMH, BMI, and HMGuptake were converted to points, and the sum of the points converted to predicted ovarian response (Figure 3).

Example 4

To explore conditions and compounds that inhibit or promote gonadotropin uptake by monocytes, cells were incubated under various conditions and were subsequently contacted with FITC-labeled gonadotropin.

Materials and methods

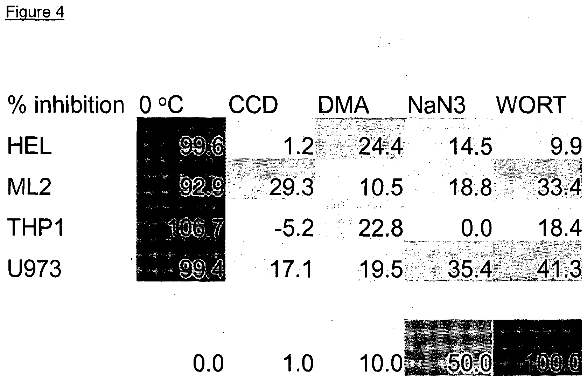

Four myelomonocytic leukemia cell lines ML2, HEL, THP-1, and U973 were propagated in cell culture media. 1 x 10$^6$ cells were pre-incubated for 2 hours at 0 °C or at 37 °C in medium supplemented with cytochalasin D, 5-N,N-dimethyl amiloride (DMA), sodium azide (NaN$_3$) or wortmannin. The cells were subsequently contacted with FITC-labeled gonadotropin for 2 hours at 0 °C or at 37 °C as appropriate, and analyzed with FACS to record FITC-specific median fluorescence intensity (MFI) was assessed in the monocyte gate. Percent inhibition of gonadotropin uptake compared to untreated cells was calculated (Figure 4).

These findings indicate that environmental cues and chemical compounds influence uptake of labeled gonadotropin by monocytes. The cues and compounds may be useful as negative control reagents to inhibit gonadotropin uptake *in vitro.* The cell-based assay described in this Example also illustrates a way to effectively screen multiple compounds affecting gonadotropin internalization by monocytes.

**Claims**

1. A method for predicting the ovarian response of a subject to ovarian stimulation with a gonadotropin, said method comprising:

   (i) contacting monocyte or macrophage cells from said subject *in vitro* with a gonadotropin; and
   (ii) assessing uptake of said gonadotropin into said cells;

   wherein uptake of gonadotropin into said cells is indicative of reduced ovarian stimulation.

2. The method of claim 1, wherein increased uptake of gonadotropin into said cells is indicative of reduced ovarian stimulation.

3. The method of claim 2, wherein said method is used to determine the dosage of a gonadotropin to administer for ovarian stimulation of a subject.

4. A method for determining a dosage of a gonadotropin to be administered to a subject for ovarian stimulation of said subject, said method comprising:

   (i) contacting monocyte or macrophage cells from said subject *in vitro* with a gonadotropin:
   (ii) assessing uptake of said gonadotropin into said cells; and
   (iii) determining said dosage from the degree of uptake.

5. The method of claim 4, wherein uptake, particularly increased uptake, of gonadotropin into said cells is indicative of decreased availability of said gonadotropin to the ovary(ies) and hence of a requirement for an increased dosage.

**6.** The method of claim 4 or claim 5, wherein in step (iii) the degree of uptake is correlated to a dosage.

**7.** The method of any one of claims 1 to 6, wherein the subject is a human.

**8.** The method of any one of claims 1 to 7, wherein the gonadotropin is FSH.

**9.** The method of any one of claims 1 to 8, wherein the cells are monocyte cells obtained or derived from blood.

**10.** The method of any one of claims 1 to 9, wherein the gonadotropin is labelled.

**11.** The method of claim 10, wherein the gonadotropin is labelled with a fluorescent or radioisotopic label, or with biotin.

**12.** A kit for predicting the ovarian response of a subject to ovarian stimulation with a gonadotropin or for determining the effect of an agent on gonadotropin activity, said kit comprising:

 (i) a gonadotropin; and at least one of
 (ii) means for assessing uptake of said gonadotropin into monocyte or macrophage cells; or
 (iii) means for isolating macrophage or monocyte cells from a subject; or
 (iv) monocyte or macrophage test cells, and

wherein said kit further comprises (v) means for determining the dosage of gonadotropin to be administered for ovarian stimulation of said subject from the degree of uptake.

**13.** The kit of claim 12, wherein the means for determining the dosage of gonadotropin to be administered for ovarian stimulation of said subject is a reference chart which correlates degree of uptake to dosage.

**14.** The kit of claim 12 or claim 13 further comprising a means for comparing the level of uptake of gonadotropin in the presence of a test agent with a reference value.

**15.** The kit of claim 14, wherein said means for comparing the level of uptake of gonadotropin in the presence of a test agent with a reference value is a reference chart which correlates the effect of the test agent on gonadotropin uptake to an effect on the activity of the gonadotropin.

**Patentansprüche**

**1.** Verfahren zum Vorhersagen der ovariellen Reaktion eines Objektes auf ovarielle Stimulation mit einem Gonadotropin, wobei das Verfahren umfasst:

 (i) Kontaktieren von Monozyten- oder Makrophagenzellen von dem Objekt in vitro mit einem Gonadotropin; und
 (ii) Beurteilen der Aufnahme des Gonadotropins in die Zellen;

wobei die Aufnahme von Gonadotropin in die Zellen indikativ für eine reduzierte ovarielle Stimulation ist.

**2.** Verfahren nach Anspruch 1, wobei die erhöhte Aufnahme von Gonadotropin in die Zellen indikativ für eine reduzierte ovarielle Stimulation ist.

**3.** Verfahren nach Anspruch 2, wobei das Verfahren verwendet wird zum Bestimmen der Dosis eines Gonadotropins, die für die ovarielle Stimulation eines Objektes verabreicht werden soll.

**4.** Verfahren zum Bestimmen einer Dosis eines Gonadotropins, die einem Objekt zur ovariellen Stimulation des Objektes verabreicht werden soll, wobei das Verfahren umfasst:

 (i) Kontaktieren von Monozyten- oder Makrophagenzellen von dem Objekt in vitro mit einem Gonadotropin:
 (ii) Beurteilen der Aufnahme des Gonadotropins in die Zellen; und
 (iii) Bestimmen der Dosis aus dem Grad der Aufnahme.

**5.** Verfahren nach Anspruch 4, wobei die Aufnahme, insbesondere eine erhöhte Aufnahme, von Gonadotropin in die Zellen indikativ für eine verringerte Verfügbarkeit des Gonadotropins für das Ovar/die Ovarien und daher für eine Notwendigkeit einer erhöhte Dosis ist.

**6.** Verfahren nach Anspruch 4 oder 5, wobei in Schritt (iii) der Grad der Aufnahme mit einer Dosis korreliert.

**7.** Verfahren nach einem der Ansprüche 1 bis 6, wobei das Objekt ein Mensch ist.

**8.** Verfahren nach einem der Ansprüche 1 bis 7, wobei das Gonadotropin FSH ist.

**9.** Verfahren nach einem der Ansprüche 1 bis 8, wobei die Zellen Monozyten-Zellen sind, die aus Blut erhalten oder abgeleitet sind.

**10.** Verfahren nach einem der Ansprüche 1 bis 9, wobei das Gonadotropin markiert ist.

**11.** Verfahren nach Einspruch 10, wobei das Gonadotropin mit einer fluoreszierenden oder Radioisotopenmarkierung oder mit Biotin markiert ist.

**12.** Set zum Vorhersagen der ovariellen Reaktion eines Objektes auf ovarielle Stimulation mit einem Gonadotropin oder zum Bestimmen des Effektes eines Mittels auf Gonadotropinaktivität, wobei das Set umfasst:

(i) ein Gonadotropin; und mindestens ein Element von folgenden:
(ii) Mittel zum Beurteilen der Aufnahme des Gonadotropins in Monozyten- oder Makrophagenzellen; oder
(iii) Mittel zum Isolieren von Makrophagen- oder Monozytenzellen von einem Objekt; oder
(iv) Monozyten- oder Makrophagen-Testzellen; und

wobei das Set ferner umfasst: (V) Mittel zum Bestimmen der Dosis von Gonadotropin, die zur ovariellen Stimulation des Objektes verabreicht werden soll, aus dem Grad der Aufnahme.

**13.** Set nach Anspruch 12, wobei das Mittel zum Bestimmen der Dosis von Gonadotropin, die zur ovariellen Stimulation des Objektes verabreicht werden soll, eine Referenztafel ist, die den Grad der Aufnahme mit der Dosis korreliert.

**14.** Set nach Anspruch 12 oder 13, das ferner ein Mittel zum Vergleichen des Grades der Aufnahme von Gonadotropin in Gegenwart eines Testmittels mit einem Referenzwert umfasst.

**15.** Set nach Anspruch 14, wobei das Mittel zum Vergleichen des Grades der Aufnahme von Gonadotropin in Gegenwart eines Testmittels mit einem Referenzwert eine Referenztafel ist, die den Effekt des Testmittels auf die Gonadotropinaufnahme mit einem Effekt auf die Aktivität des Gonadotropins korreliert.

**Revendications**

**1.** Procédé de prédiction de la réponse ovarienne d'un sujet à une stimulation ovarienne avec une gonadotrophine, ledit procédé comprenant :

(i) une mise en contact de cellules monocytes ou macrophages dudit sujet *in vitro* avec une gonadotrophine ; et
(ii) une évaluation de l'absorption de ladite gonadotrophine dans lesdites cellules ;

dans lequel l'absorption de gonadotrophine dans lesdites cellules est révélatrice d'une stimulation ovarienne réduite.

**2.** Procédé selon la revendication 1, dans lequel l'absorption accrue de gonadotrophine dans lesdites cellules est révélatrice d'une stimulation ovarienne réduite.

**3.** Procédé selon la revendication 2, dans lequel ledit procédé est utilisé pour déterminer le dosage d'une gonadotrophine à administrer pour une stimulation ovarienne d'un sujet.

**4.** Procédé de détermination d'un dosage d'une gonadotrophine à administrer à un sujet pour une stimulation ovarienne dudit sujet, ledit procédé comprenant :

(i) une mise en contact de cellules monocytes ou macrophages dudit sujet *in vitro* avec une gonadotrophine ;
(ii) une évaluation de l'absorption de ladite gonadotrophine dans lesdites cellules ; et
(iii) une détermination dudit dosage à partir du degré d'absorption.

**5.** Procédé selon la revendication 4, dans lequel l'absorption, en particulier l'absorption accrue, de gonadotrophine dans lesdites cellules est révélatrice d'une disponibilité réduite de ladite gonadotrophine pour l'ovaire ou les ovaires et par conséquent d'une nécessité d'un dosage accru.

**6.** Procédé selon la revendication 4 ou la revendication 5, dans lequel dans l'étape (iii) le degré d'absorption est corrélé à un dosage.

**7.** Procédé selon l'une quelconque des revendications 1 à 6, dans lequel le sujet est un humain.

**8.** Procédé selon l'une quelconque des revendications 1 à 7, dans lequel la gonadotrophine est de la FSH.

**9.** Procédé selon l'une quelconque des revendications 1 à 8, dans lequel les cellules sont des cellules monocytes obtenues ou dérivées du sang.

**10.** Procédé selon l'une quelconque des revendications 1 à 9, dans lequel la gonadotrophine est marquée.

**11.** Procédé selon la revendication 10, dans lequel la gonadotrophine est marquée avec un marqueur fluorescent ou radio-isotopique, ou avec de la biotine.

**12.** Kit de prédiction de la réponse ovarienne d'un sujet à une stimulation ovarienne avec une gonadotrophine ou de détermination de l'effet d'un agent sur l'activité de gonadotrophine, ledit kit comprenant :

(i) une gonadotrophine ; et au moins un parmi

(ii) des moyens d'évaluation de l'absorption de ladite gonadotrophine dans des cellules monocytes ou macrophages ; ou

(iii) des moyens d'isolement de cellules ou macrophages ou monocytes d'un sujet ; ou

(iv) des cellules de test monocytes ou macrophages ; et

dans lequel ledit kit comprend en outre (v) des moyens de détermination du dosage de gonadotrophine à administrer pour une stimulation ovarienne dudit sujet à partir du degré d'absorption.

13. Kit selon la revendication 12, dans lequel les moyens de détermination du dosage de gonadotrophine à administrer pour une stimulation ovarienne dudit sujet sont un tableau de référence qui corrèle le degré d'absorption au dosage.

14. Kit selon la revendication 12 ou la revendication 13 comprenant en outre un moyen de comparaison du niveau d'absorption de gonadotrophine en présence d'un agent de test avec une valeur de référence.

15. Kit selon la revendication 14, dans lequel ledit moyen de comparaison du niveau d'absorption de gonadotrophine en présence d'un agent de test avec une valeur de référence est un tableau de référence qui corrèle l'effet de l'agent de test sur l'absorption de gonadotrophine à un effet sur l'activité de la gonadotrophine.

Figure 1

Figure 1 (contd.)

C gonadotropin     dextran     nucleus

D granulosa cell     + monocytes for 24h
  gonadotropin

granulosa cell     granulosa cell     monocytes

Figure 2

A     HMG-FITC

negative control (PBS)

CD14-PerCP

HMG-FITC

Figure 2 (contd.)

Figure 3

Points

AMH (pmol/l)

BMI (kg/m²)

HMG uptake

Sum of points

Predicted ovarian response

low ... high

Figure 4

| % inhibition | 0 °C | CCD | DMA | NaN3 | WORT |
|---|---|---|---|---|---|
| HEL | 99.6 | 1.2 | 24.4 | 14.5 | 9.9 |
| ML2 | 92.9 | 29.3 | 10.5 | 18.8 | 33.4 |
| THP1 | 106.7 | -5.2 | 22.8 | 0.0 | 18.4 |
| U973 | 99.4 | 17.1 | 19.5 | 35.4 | 41.3 |

| 0.0 | 1.0 | 10.0 | 50.0 | 100.0 |
|---|---|---|---|---|

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FAUSER et al.** *Hum Reprod Update,* 2008, vol. 14, 1-14
- **BROER et al.** *Human Reproduction Update,* 2011, vol. 17 (1), 46-54
- **AL-AZEMI et al.** *Human Reproduction,* 2011, vol. 26, 414-422
- **ALTMÄE et al.** *Human Reproduction Update,* 2011, vol. 17, 813-828
- **KATABUCHI ; OHBA.** *Develop. Growth Differ.,* 2008, vol. 50, 299-306